# FASCICULE DE BREVET EUROPEEN

(11) **EP 4 311 767 B1**
(45) Date de publication et mention de la délivrance du brevet: **16.04.2025**
(21) Numéro de dépôt: 23187459.5
(22) Date de dépôt: 25.07.2023
(51) Int. Cl.: B64C 13/04, B64C 13/10, A61B 5/18

(54) **SYSTÈME DE SURVEILLANCE D'UN PILOTE D'UN AÉRONEF POUR DÉTECTER UNE INCAPACITATION DU PILOTE ; PROCÉDÉ ET PRODUIT PROGRAMME D ORDINATEUR ASSOCIÉS**
SYSTEM ZUR ÜBERWACHUNG EINES PILOTEN EINES LUFTFAHRZEUGS ZUR ERKENNUNG EINER PILOTENUNREGELMÄSSIGKEIT; VERFAHREN UND COMPUTERPROGRAMMPRODUKT DAFÜR
SYSTEM FOR MONITORING A PILOT OF AN AIRCRAFT FOR DETECTING A PILOT INCAPACITATION; ASSOCIATED METHOD AND COMPUTER PROGRAM PRODUCT

(30) Priorité: 26.07.2022 FR 2207666
(43) Date de publication de la demande: 31.01.2024
(73) Titulaire: THALES, 92400 Courbevoie (FR)
(72) Inventeur: BECOUARN, Loïc, 33700 MERIGNAC (FR); BERTHELOT, Bastien, 33700 MERIGNAC (FR); SARRAUD, Clément, 33320 EYSINES (FR); IBANEZ, Vincent, 33700 MERIGNAC (FR)
(74) Mandataire: Lavoix

(56) Documents cités:
- EP-A1- 4 092 509
- WO-A1-2022/080512
- DE-U1- 202007 015 835
- FR-A1- 3 092 564
- US-A1- 2021 034 053
- US-B2- 10 426 393

## Description

La présente invention concerne le domaine technique des systèmes et des procédés de surveillance du pilote d'un aéronef.

Certains acteurs de l'aéronautique civile envisagent de faire évoluer le contexte opérationnel de certains aéronefs de manière à ce que l'équipage soit réduit à un unique pilote pendant au moins une partie du vol, le pilote se retrouvant alors seul dans le cockpit.

Cependant, ceci impose de mettre en œuvre des procédures de surveillance du pilote afin de garantir la sécurité du vol.

Un certain nombre de procédures de surveillance ont été proposées utilisant des capteurs physiologiques portés par le pilote afin de déterminer certains états du pilote, comme son niveau de fatigue, sa charge de travail, son niveau de stress, etc.

Par exemple, le document EP3154038 divulgue un procédé d'évaluation de l'état de fatigue d'un pilote à partir de la détection des mouvements des organes de pilotage.

Mais ces procédures de surveillance ne permettent pas d'anticiper une incapacitation du pilote. En effet, en cas d'incapacitation, le pilote est incapable non seulement de mener à bien sa mission, mais également de le déclarer. C'est le cas par exemple lors d'une perte de connaissance suite à un malaise vagal ou à une crise cardiaque.

Il est à souligner qu'état de fatigue et incapacitation ne sont pas synonymes. Ainsi, s'il est possible d'utiliser des capteurs de détection des mouvements des organes de pilotage pour déterminer l'état de fatigue du pilote, de tels capteurs ne permettent pas de déterminer une incapacitation du pilote.

Il a donc été proposé de faire appel à un autre type de système de surveillance pour détecter une incapacitation.

Le document FR3098334A1 divulgue un système de surveillance de l'état de conscience du pilote fondé sur l'acquisition d'images et de leur traitement afin de détecter les mouvements oculaires du pilote. Si aucun mouvement n'est détecté, un signal de perte de connaissance est émis.

Les documents FR 3 092 564 A1, DE 20 2007 015835 U1, WO 2022/080512 A1, US 2021/034053 A1 et US 10 426 393 B2 divulguent d'autres systèmes de surveillance d'un pilote.

On notera qu'état de conscience et incapacitation sont synonymes et l'utilisation d'images du pilote pour déterminer l'état de conscience pourrait aussi permettre de déterminer une incapacitation du pilote.

Cependant, un tel système de surveillance ne permettrait pas de détecter une incapacitation du pilote très rapidement, ce qui est indispensable dans certaines phases de vol (comme la phase de roulage) qui doivent rester sous le contrôle manuel du pilote. Ainsi, toute incapacitation du pilote doit pouvoir être détectée quasi-immédiatement, typiquement en moins d'une demie seconde, pour initier les contre-mesures adaptées.

Or, une détection fondée sur les mouvements oculaires du pilote ne permet pas d'atteindre une telle rapidité de détection, ne serait-ce que par le temps de traitement de chaque image et la nécessité d'acquérir une succession temporelle d'images pour, en les comparant, déterminer l'état du pilote.

Le but de la présente invention est par conséquent de résoudre ce problème, en proposant notamment un système de surveillance permettant de déclencher une alerte avec une très faible latence lors d'une incapacitation du pilote.

Pour cela l'invention a pour objet un système de surveillance d'un pilote d'un aéronef conforme aux revendications annexées.

L'invention et ses avantages seront mieux compris à la lecture de la description détaillée qui va suivre d'un mode de réalisation particulier, donné uniquement à titre d'exemple non limitatif, cette description étant faite en se référant aux dessins annexés sur lesquels :
[Fig 1] La figure 1 est une représentation schématique d'un mode de réalisation du système de surveillance selon l'invention ;
[Fig 2] La figure 2 est une représentation schématique d'un mode de réalisation du procédé de surveillance mis en œuvre dans le système de la figure 1 ; et,
[Fig 3] La figure 3 est une représentation sous forme de blocs d'un algorithme de génération d'alarme du procédé de la figure 2.

Le système de surveillance du pilote d'un aéronef est destiné à être embarqué à bord du cockpit d'un aéronef (comme un avion, un hélicoptère, ou l'équivalent) ou d'un poste de pilotage d'un drone, afin de détecter l'occurrence d'une incapacitation du pilote en temps réel (moins d'une demie seconde).

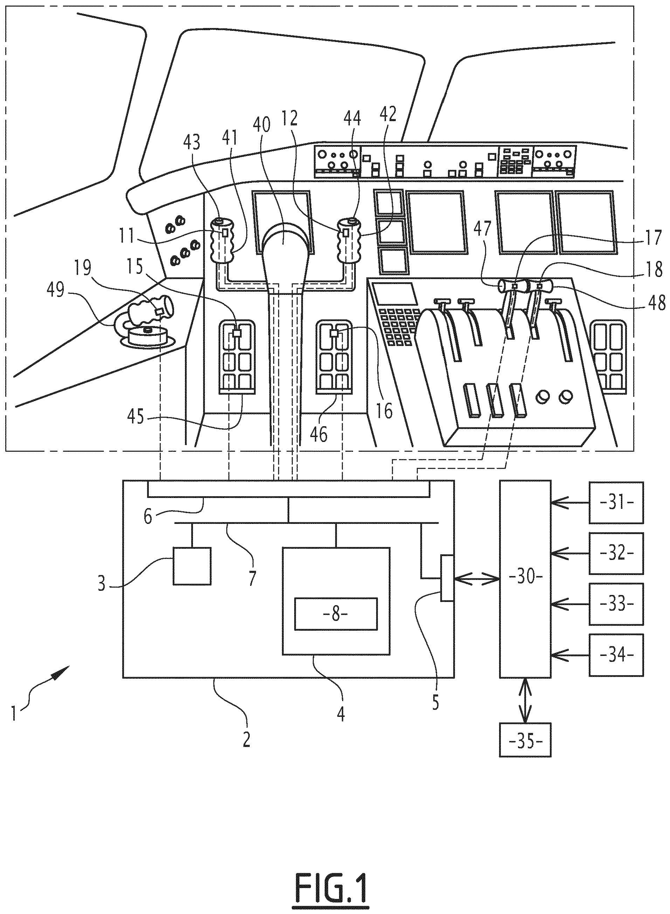

Comme l'illustre le mode de réalisation de la figure 1, le système de surveillance 1 comporte une unité de calcul 2 et une pluralité de capteurs de contact 11 à 19 connectés en entrée de l'unité de calcul 2.

L'unité de calcul 2 est un ordinateur comportant des moyens de calcul, tels qu'un processeur 3, des moyens de mémorisation, tels qu'une mémoire 4, un ensemble de connecteurs 6, permettant de connecter chaque capteur à l'unité de calcul 2, une interface réseau 5, permettant des communications bidirectionnelles sur un réseau 30 de l'aéronef, et un bus de communication interne 7, reliant les différents composants de l'unité de calcul 2.

La mémoire 4 stocke les instructions de programmes d'ordinateurs, en particulier celles d'un programme de surveillance 8 dont l'exécution permet la mise en œuvre d'un procédé de surveillance du pilote, qui est fondé sur la génération d'une alarme lorsqu'une incapacitation du pilote est détecté à partir des signaux délivrés par tout ou partie des capteurs de contact.

L'unité de calcul 2 est interfacée avec le réseau 30 de l'aéronef. Le format des communications sur ce réseau dépend du fabricant de l'aéronef et l'unité de calcul est donc adaptée pour émettre et recevoir des messages au format requis (AFDX, ARINC, etc.), en particulier pour émettre des alarmes au format requis.

A ce réseau 30 sont également connectés différents systèmes annexes capables de fournir des informations de contexte de vol, Info_Context, à l'unité de calcul 2. Par exemple, un système de pilotage automatique 31 indique l'état d'engagement du pilote automatique ; un système de freinage parking 32 indique l'état d'application du frein de parking ; un système de contrôle de vol 33 indique la phase de vol courante de l'aéronef ; et un système d'instrumentation 34 indique un ou plusieurs paramètres de vol, comme la hauteur courante de l'aéronef ou sa vitesse sol.

Et également connecté au réseau de l'avion, un système de gestion d'alarmes 35. Lorsque l'unité de calcul 2 détecte l'incapacitation du pilote, il émet une alarme sur le réseau 30 à destination du système 35. En fonction de cette alarme et de la situation opérationnelle, le système 35 est capable d'engager les contre-mesures nécessaires permettant de garantir la sécurité de l'aéronef.

Chaque capteur de contact est monté en surface sur un organe de pilotage associé.

Un capteur de contact est capable de détecter une pression exercée par le pilote sur l'organe de pilotage qu'il équipe et de générer un signal à destination de l'unité de calcul 2, le signal correspondant à une mesure d'une pression exercée par le pilote sur l'organe de pilotage associé.

Un organe de pilotage équipé d'un capteur de contact est tel que le pilote doit exercer une certaine pression sur l'organe de pilotage s'il veut le manipuler correctement pour piloter l'aéronef convenablement. Les organes de pilotage équipés de capteurs de contact sont donc les organes de pilotage actionnables par application d'une force par le pilote. Bien que cette force puisse être appliquée avec le pied ou la main, on parlera d'organes de pilotage actionnables « manuellement » dans ce qui suit.

Par exemple, dans le mode de réalisation illustré sur la figure 1, un premier capteur de contact gauche 11 et un premier capteur de contact droit 12 sont disposés sur le manche 40 de l'aéronef (respectivement sur la poignée gauche 41 et sur la poignée droite 42 du manche). A noter que l'invention n'est pas spécifique d'un type de manche (ou volant) et peut être mise en œuvre que le manche soit du type « disposé devant le pilote » (comme illustré sur la figure 1) ou du type « disposé sur le côté » (mini manche à commandes électriques).

Par exemple encore, un second capteur de contact gauche 15 et un second capteur de contact droit 16 sont disposés sur les pédales de palonnier, respectivement gauche 45 et droite 46.

Par exemple encore, un troisième capteur de contact gauche 17 et un troisième capteur de contact droit 18 sont disposés sur la manette des gaz du moteur gauche 47 et celle du moteur droit 48 respectivement.

Par exemple encore, un cinquième capteur de contact 19 équipe avantageusement la manette de roulette de nez 49.

En variante, d'autres organes de pilotage pourraient porter un capteur de contact, comme une manette de réglage du train d'atterrissage, un interrupteur ou un écran du tableau de bord, un outil de saisie, comme une souris ou une boule de commande (« trackball » en anglais), etc.

Les capteurs de contact peuvent être :
- des capteurs mécaniques, par exemple à contact électrique (le contact de la main du pilote sur une partie mobile du capteur équipant l'organe de pilotage génère une action mécanique permettant d'établir ou d'interrompre un contact électrique) ;
- des capteurs de pression, par exemple piézoélectriques (notamment piézorésistifs), résistifs ou capacitifs (le contact de la main du pilote sur une surface du capteur équipant l'organe de pilotage génère une déformation du capteur à l'origine d'une modification d'une propriété électrique du capteur) ; ou encore,
- des capteurs de proximité, par exemple à infrarouge (le contact de la main du pilote sur le capteur équipant l'organe de pilotage permet de réfléchir un signal de détection).

Différentes méthodes pour intégrer un capteur de contact sur un organe de pilotage sont connues de l'homme de métier.

Selon l'invention revendiquée, sont mis en œuvre des capteurs de contact du type capacitif, car leur intégration est plus simple. On peut par exemple citer la possibilité de réaliser un capteur capacitifs (et le circuit associé) par impression 3D sur la surface externe de l'organe de pilotage. Ceci est particulièrement intéressant pour équiper les organes de pilotage ergonomiques, comme les poignées ou les manettes.

Les capteurs capacitifs sont plus adaptés à une intégration sur des commandes de vol puisque qu'ils sont très fins, invisibles pour le pilote et nécessitent une électronique de contrôle relativement simple.

De plus, les capteurs du type bouton poussoir ne permettent pas de couvrir toutes les positions possibles de la main du pilote sur un organe de contrôle. En particulier, la main peut avoir des positions très variables sur la manette des gaz entre la position « plein gaz » (manettes vers l'avant) à la position « inversion de poussée » (manette au maximum arrière). En revanche, il est possible de couvrir de manière large la surface d'un organe de contrôle, comme la manette des gaz, avec un capteur capacitif et ainsi détecter l'activité du pilote quelle que soit la position de cet organe de contrôle.

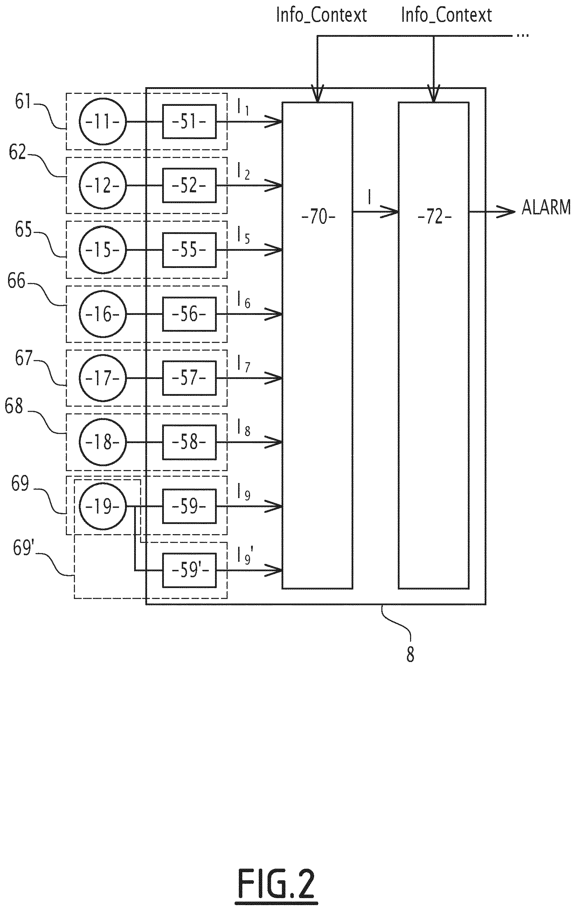

Comme représenté schématiquement sur la figure 2, l'exécution du programme 8 permet de doter l'unité de calcul 2 de différentes fonctionnalités, notamment une pluralité de modules de modélisation 51 à 59, un module d'agrégation 70 et un module de génération d'alarme 72.

Chaque capteur de contact 11 à 19 est associé à au moins un module de modélisation 51 à 59.

Un capteur de contact et le module de modélisation associé forment ensemble un détecteur de pression.

Le module de modélisation est capable d'acquérir le signal généré par le capteur associé et de déterminer la valeur d'un indicateur élémentaire qui indique si le pilote exerce sur l'organe de pilotage correspondant une pression adaptée.

Le module de modélisation met ainsi en œuvre un modèle de contact qui dépend de la nature de l'organe de pilotage correspondant, c'est-à-dire de la manière dont cet organe doit être manipulé par le pilote.

Par exemple, le modèle de modélisation compare la pression mesurée par le capteur de contact associé à un seuil de pression et un indicateur est émis lorsque la pression exercée est supérieure à ce seuil.

En variante, le modèle de contact prend en compte la phase de vol pour sélectionner le modèle de contact qu'il convient de mettre en œuvre. Par exemple, la phase de vol peut permettre de définir la valeur du seuil de pression à prendre en compte.

La détection que le pilote applique la pression requise sur l'organe de pilotage est indicative d'une action intentionnelle du pilote et par conséquent qu'il n'est pas en incapacitation. L'invention est donc fondée sur l'hypothèse qu'en cas de perte de connaissance du pilote, celui-ci ne sera plus en mesure d'appliquer sur l'organe de pilotage la pression normalement requise pour contrôler cet équipement.

La pluralité de détecteurs de pression comporte au moins un détecteur de pression de contact (ou détecteur de contact dans ce qui suit). Un détecteur de contact doit permettre de détecter une pression faible exercée par le pilote sur l'organe de pilotage correspondant (par exemple le modèle mis en œuvre utilise un seuil de pression bas).

La pluralité de détecteurs de pression comporte avantageusement au moins un détecteur de pression de force (ou détecteur de force) dans ce qui suit. Un détecteur de force doit permettre de détecter une pression élevée exercée par le pilote sur l'organe de pilotage correspondant (par exemple le modèle mis en œuvre utilise un seuil de pression haut).

Par exemple, les premiers détecteurs 61, 62 intégrant les premiers capteurs 11 et 12 qui équipent le manche 40 sont du type détecteurs de contact : lorsque le pilote saisit correctement la poignée gauche dans sa main gauche, il applique une pression détectable par le premier détecteur droit et lorsque le pilote saisit correctement la poignée droite dans sa main droite, il exerce une pression détectable par le premier détecteur droit. Ces premiers détecteurs permettent donc de générer les indicateurs élémentaires I1, I2 indiquant le simple fait que le pilote a la main correctement posée sur la poignée.

Par exemple, les seconds détecteurs 65, 66 intégrant les seconds capteurs 15 et 16 qui équipent les pédales du palonnier sont du type détecteurs de contact : pour faire tourner l'avion en phase de roulage au sol ou lui faire faire un virage en l'air, le pilote pose les pieds sur chaque pédale. Ces seconds détecteurs permettent donc de générer les indicateurs I5, I6 indiquant que le pilote a les pieds correctement posés sur les pédales.

Par exemple, les troisièmes détecteurs 67, 68 intégrant les troisième capteurs 17 et 18 qui équipent les manettes des gaz sont du type détecteurs de contact : lorsque le pilote saisit correctement l'une et/ou l'autre de ces manettes, sans pour autant les déplacer, il applique une pression détectable. Des indicateurs élémentaires I7 et I8 sont générés.

Par exemple encore, le détecteur 69 intégrant le capteur 19 équipant la manette de roulette de nez est associé à un premier module de modélisation 59 qui utilise un modèle « en ligne droite » permettant de détecter que le pilote a la main posée sur cette manette. Il s'agit encore d'un détecteur de contact. Il émet un indicateur élémentaire I9.

Un exemple de capteur de force est donné par le détecteur 69' intégrant le capteur 19 équipant la manette de roulette de nez et un second module de modélisation 59' qui utilise un modèle « en virage » imposant que le pilote exerce une pression élevée permettant de s'opposer à la force de rappel forçant la manette à revenir en position neutre. Il s'agit d'un détecteur de force. Il émet un indicateur élémentaire I9'.

Un détecteur permet donc de déterminer si une action intentionnelle particulière est réalisée par le pilote sur l'organe de pilotage correspondant et, dans l'affirmative, émettre l'indicateur élémentaire associé.

Il est à noter qu'un capteur de contact, par exemple capacitif, pourrait permettre la détection d'une pression basse sans avoir besoin d'y adjoindre un module de modélisation. Cependant, le capteur de contact étant monté sur une surface courbe (comme celle de la poignée du manche), il peut être soumis à des contraintes mécaniques qui conduisent à l'émission par le capteur de contact d'un signal correspondant à l'application d'une pression alors même qu'aucune pression n'est effectivement exercée par le pilote sur le capteur. Le module de modélisation permet alors de calibrer le capteur de contact, en ajustant par exemple le seuil de pression au-delà de laquelle une information de détection est émise.

Le module d'agrégation 70 est propre à agréger tout ou partie des indicateurs élémentaires à l'instant courant pour déterminer un état courant du pilote. Par exemple, l'état courant du pilote est un indicateur global I prenant la valeur unité lorsqu'il est détecté que le pilote réalise une action intentionnelle ou la valeur nulle lorsqu'il est détecté aucune action intentionnelle du pilote, c'est-à-dire que le pilote est en incapacitation. Dit autrement, une valeur nulle de l'indicateur global correspond à la détection d'une incapacitation du pilote.

Le module d'agrégation 70 prend avantageusement en compte les informations de contexte de vol, Info_Context, pour sélectionner les indicateurs élémentaires à agréger pour calculer l'indicateur global. Une telle information de contexte de vol provient de l'un ou l'autre des systèmes annexes 31 à 34.

Par exemple, si l'unité de calcul 2 reçoit une information de contexte de vol selon laquelle l'aéronef est en phase de croisière et que le pilote automatique est engagé, cela signifie que le pilote n'a aucune raison de tenir le manche 40. En conséquence, il convient d'ignorer les indicateurs élémentaires dérivés des signaux provenant des capteurs de contact disposés sur les organes de commandes de vol, comme les capteurs 11 et 12.

Enfin, le module de génération d'alarmes 72 est capable de générer une alarme et de la transmettre au système de gestion d'alarmes 35. Le module 72 exécute un algorithme qui prend en entrée l'indicateur global I à l'instant courant pour décider de l'émission d'une alarme. De préférence, cet algorithme utilise également des informations de contexte, Info_Context.

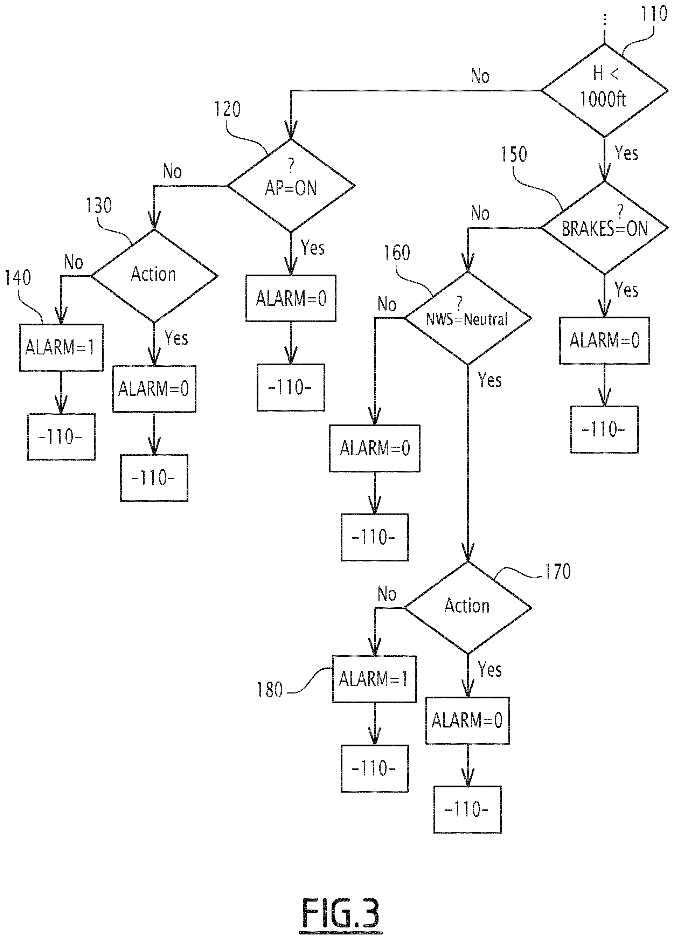

Par exemple, comme représenté sur la figure 3, l'algorithme, qui est mis en œuvre par le module 72, compare (étape 110) la hauteur de vol H à un seuil prédéfini, valant par exemple 1000 pieds.

Lorsque la hauteur est supérieure ou égale à ce seuil, l'algorithme vérifie (étape 120) si le pilote automatique - AP (« AutoPilot » en anglais) est engagé (état « ON »).

Dans l'affirmative, l'algorithme éteint l'alarme existante, s'il y en avait une (par exemple en attribuant la valeur nulle à la variable ALARM), et boucle sur l'étape 110.

Dans la négative, l'algorithme vérifie (étape 130) la valeur de l'indicateur global I pour savoir si le pilote effectue une action intentionnelle.

Dans l'affirmative, l'algorithme supprime l'alarme existante, s'il y en avait une, et boucle sur l'étape 110.

Dans la négative, une incapacitation du pilote est détectée et une alarme est émise (étape 140). Par exemple la valeur unité est attribuée à la variable ALARM. L'algorithme boucle ensuite sur l'étape 110 pour une nouvelle itération.

En revanche, lorsque la hauteur H est inférieure au seuil de 1000 pieds, l'algorithme vérifie (étape 150) si le frein de parking (« Brakes ») est appliqué (état « ON »).

Dans l'affirmative, l'algorithme supprime l'alarme existante, s'il y en avait une, et boucle sur l'étape 110.

Dans la négative, l'algorithme vérifie (étape 160) si la manette de roulette de nez - NWS (« Nose Wheel Steering ») est en position neutre (état « Neutral »).

Dans la négative, l'algorithme supprime l'alarme existante, s'il y en avait une, et boucle sur l'étape 110.

Dans l'affirmative, l'algorithme vérifie (étape 170) la valeur de l'indicateur global I pour savoir si le pilote effectue une action intentionnelle.

Dans l'affirmative, l'algorithme supprime l'alarme existante, s'il y en avait une, et boucle sur l'étape 110.

Dans la négative, une incapacitation du pilote est détectée et une alarme est émise (étape 180). L'algorithme boucle sur l'étape 110 pour une nouvelle itération.

Le futur mode opératoire des aéronefs lorsque le pilote se retrouvera seul à bord en phase d'atterrissage pourrait imposer d'avoir les mains sur le manche de manière à être prêt à réagir lorsque la hauteur de l'avion est inférieure à une certaine valeur, ici prise par exemple à 1000 pieds. En conséquence, si les capteurs de contact disposés sur le manche ou la manette des gaz ne détectent aucune pression (seuls capteurs pris en compte dans cette situation pour calculer l'indicateur global), une alarme est émise immédiatement.

Cette contrainte d'avoir les mains sur le manche n'est pas stricte au-dessus des 1000 pieds et les indicateurs élémentaires dérivant des capteurs de contact du manche ne suffisent pas pour détecter une incapacitation.

Lors de la réception d'une alarme, le système de gestion d'alertes 35 peut commencer par émettre une alerte sonore dans le cockpit et attendre de l'unité de calcul 2 une suspension de l'alarme précédemment déclenchée, indiquant ainsi que le pilote a repris le contrôle de l'aéronef, par exemple en replaçant immédiatement ses mains sur le manche au-dessous du plafond des 1000 pieds.

En revanche, si pendant un intervalle de temps prédéterminé, l'unité de calcul 2 continue à émettre une alarme, le système 35 passe à des contre-mesures pour prendre la main sur le pilotage de l'aéronef, par exemple en engageant le pilote automatique.

Il est à noter que la figure 2 pourrait être lue comme les différentes étapes d'un procédé de surveillance. Selon ce procédé, l'unité de calcul 2 réalise :
- l'acquisition des signaux délivrés par les différents capteurs de contact,
- le traitement de ces signaux afin de déterminer l'état courant du pilote : par exemple d'abord en calculant un indicateur élémentaire en vérifiant que la pression mesurée par un capteur est adaptée à l'organe de pilotage suivi (i.e. la pression mesurée est conforme à un modèle), puis en agrégeant tout ou partie de ces indicateurs élémentaires dans un indicateur global en tant qu'état du pilote ;
- et la génération d'une alarme lorsque l'état du pilote correspond à une incapacitation.

Avantageusement, les étapes de traitement du signal et de génération d'alarme prennent en compte des informations contextuelles délivrées par d'autres systèmes embarqués à bord de l'aéronef.

En variante, un détecteur de pression est un dispositif indépendant de l'unité de calcul. Cependant il est préférable que le module de modélisation associé à un capteur de contact soit du type logiciel, dans l'unité de calcul, pour pouvoir ajuster les paramètres du modèle, comme par exemple le seuil de pression.

Éventuellement, un organe de pilotage, tel qu'une poignée, peut être équipé de plusieurs capteurs de contact, disposés à des emplacements différents pour autoriser différentes positions de la main du pilote sur cette poignée. En multipliant les points de mesure ou en augmentant la surface du capteur de contact, on limite l'occurrence de fausses alarmes.

D'autres types de capteurs peuvent éventuellement être connectés à l'unité de calcul. Il peut s'agir de caméras analysant le comportement du pilote, d'accéléromètres intégrés dans le casque du pilote pour détecter les mouvements de tête, des capteurs physiologiques tels que ceux mesurant le rythme cardiaque, un micro analysant l'ambiance sonore à l'intérieur de la cabine (notamment les sons émis par les pilotes), etc.

D'autres indicateurs peuvent être dérivés soit des signaux délivrés par ces autres capteurs équipant le cockpit, soit de l'actionnement d'autres équipements du cockpit. Par exemple, comme illustré sur la figure 1, les poignées du manche 40 sont équipées de boutons gauche et droit, 43 et 44. Le bouton gauche 43 permet par exemple de désengager le pilote automatique, tandis que le bouton droit permet au pilote de transmettre sur la radio. L'actionnement de l'un ou l'autre de ces boutons peut, en variante, servir d'indicateur d'une action volontaire du pilote.

## Revendications

1. Système de surveillance (1) d'un pilote d'un aéronef, pour détecter une incapacitation du pilote, l'aéronef étant équipé d'au moins un organe de pilotage (40) actionnable manuellement par le pilote, **caractérisé en ce que** le système de surveillance (1) comporte :
- l'organe de pilotage (40),
- au moins un détecteur de pression (62, 65) de manière à générer un indicateur élémentaire lorsqu'une pression exercée par le pilote sur l'organe de pilotage est adaptée à un usage de cet organe de pilotage, le détecteur de pression intégrant :
- un capteur de pression capacitif (11, 12) monté en surface de l'organe de pilotage (40), et,
- un module de modélisation (51 à 59), le module de modélisation étant capable, à partir du signal délivré par le capteur de pression capacitif, de calculer l'indicateur élémentaire, et mettant en œuvre un modèle de contact qui dépend de la nature de l'organe de pilotage,
- une unité de calcul (2) programmée pour traiter l'indicateur élémentaire délivré par le détecteur de pression et déterminer un état courant du pilote et, lorsque ledit état courant correspond à une incapacitation du pilote, émettre une alarme (ALARM).

2. Système selon la revendication 1, dans lequel le détecteur de pression est un détecteur de pression de contact adapté pour détecter le contact de la main du pilote sur l'organe de pilotage.

3. Système selon la revendication 1 ou la revendication 2, dans lequel le détecteur de pression est un détecteur de pression de force adapté pour détecter l'application d'une force par le pilote sur l'organe de pilotage.

4. Système selon l'une quelconque des revendications 1 à 3, dans lequel l'unité de calcul (2) reçoit, d'un système annexe (31 à 34) équipant l'aéronef, au moins une information de contexte de vol (Info_Context), et dans lequel l'unité de calcul (2) traite l'indicateur élémentaire délivré par le détecteur de pression en tenant compte de ladite information de contexte de vol.

5. Système selon la revendication 4, dans lequel l'information de contexte de vol (Info_Context) est une information de phase de vol de l'aéronef ou une information sur une valeur d'au moins un paramètre de vol de l'aéronef, l'information de phase de vol étant sélectionnée parmi : une phase de roulage, une phase de décollage, une phase de croisière, et une phase d'atterrissage, et l'information de paramètre de vol étant sélectionnée parmi : une hauteur, une vitesse, un état d'engagement du pilote automatique, une position d'une manette de roulette de nez, une position d'une manette des gaz, une position d'un manche, et une position d'un palonnier.

6. Système selon l'une quelconque des revendications 1 à 5, comportant une pluralité de détecteurs de pression et un module d'agrégation (70) propre à agréger tout ou partie de la pluralité d'indicateurs délivrés par la pluralité de détecteurs de pression dans un indicateur global en tant qu'état courant du pilote.

7. Système selon la revendication 6, dans lequel le module d'agrégation (70) sélectionne les indicateurs élémentaires à prendre en compte pour calculer l'indicateur global en fonction d'une information de contexte de vol.

8. Système selon la revendication 6 ou la revendication 7, dans lequel l'unité de calcul (2) comporte un module de génération d'alarme (72) capable d'appliquer un algorithme utilisant une ou plusieurs informations de contexte de vol et la valeur de l'indicateur global déterminé par le module d'agrégation (70) pour déterminer s'il faut émettre une alarme.

## Patentansprüche

1. System zur Überwachung (1) eines Piloten eines Luftfahrzeugs zur Erkennung einer Pilotenunregelmässigkeit, wobei das Luftfahrzeug mit mindestens einem Steuerungsorgan (40) ausgestattet ist, das vom Piloten manuell betätigt werden kann, **dadurch gekennzeichnet, dass** das System zur Überwachung (1) Folgendes umfasst:
- das Steuerungsorgan (40),
- mindestens einen Druckdetektor (62, 65), um eine elementare Anzeige zu erzeugen, wenn ein Druck, der vom Piloten auf das Steuerungsorgan ausgeübt wird, an eine Verwendung dieses Steuerungsorgans angepasst ist, wobei der Druckdetektor Folgendes beinhaltet:
- einen kapazitiven Drucksensor (11, 12), der auf der Oberfläche des Steuerungsorgans (40) montiert ist, und
- ein Modellbildungsmodul (51 bis 59), wobei das Modellbildungsmodul dazu in der Lage ist, ausgehend von dem Signal, das von dem kapazitiven Drucksensor geliefert wird, die elementare Anzeige zu berechnen, und ein Kontaktmodell zu installieren, das von der Art des Steuerungsorgans abhängt,
- eine Berechnungseinheit (2), die programmiert ist, um die elementare Anzeige zu verarbeiten, die vom Druckdetektor geliefert wird, und einen aktuellen Zustand des Piloten zu bestimmen, und, wenn der aktuelle Zustand einer Handlungsunfähigkeit des Piloten entspricht, einen Alarm (ALARM) auszugeben.

2. System nach Anspruch 1, wobei der Druckdetektor ein Kontaktdruckdetektor ist, der ausgelegt ist, um den Kontakt der Hand des Piloten auf dem Steuerungsorgan nachzuweisen.

3. System nach Anspruch 1 oder Anspruch 2, wobei der Druckdetektor ein Kraftdruckdetektor ist, der ausgelegt ist, um die Anwendung einer Kraft durch den Piloten auf das Steuerungsorgan nachzuweisen.

4. System nach einem der Ansprüche 1 bis 3, wobei die Berechnungseinheit (2) von einem beigefügten System (31 bis 34), mit dem das Luftfahrzeug ausgestattet ist, mindestens eine Flugkontextinformation (Info_Context) empfängt, und wobei die Berechnungseinheit (2) die elementare Anzeige, die von dem Druckdetektor geliefert wurde, verarbeitet, indem sie die Flugkontextinformation berücksichtigt.

5. System nach Anspruch 4, wobei die Flugkontextinformation (Info_Context) eine Flugfaseninformation des Luftfahrzeugs oder eine Information über einen Wert mindestens eines Flugparameters des Luftfahrzeugs ist, wobei die Flugfaseninformation ausgewählt ist aus: einer Rollfase, einer Startfase, einer Flugfase und einer Landefase, und wobei die Flugparameterinformation ausgewählt ist aus: einer Höhe, einer Geschwindigkeit, einem Zustand des Eingreifens des Autopiloten, einer Position eines Bugrad-Steuerknüppels, einer Position eines Gashebels, einer Position eines Steuerknüppels und einer Position eines Seitenruderpedals.

6. System nach einem der Ansprüche 1 bis 5, umfassend eine Vielzahl von Druckdetektoren und ein Aggregationsmodul (70), das geeignet ist, um die Gesamtheit oder einen Teil der Vielzahl von Anzeigen zu aggregieren, die von der Vielzahl von Druckdetektoren in einer globalen Anzeige als der aktuelle Zustand des Piloten geliefert werden.

7. System nach Anspruch 6, wobei das Aggregationsmodul (70) die elementaren Anzeigen auswählt, die berücksichtigt werden sollen, um die globale Anzeige in Funktion einer Flugkontextinformation zu berechnen.

8. System nach Anspruch 6 oder Anspruch 7, wobei die Berechnungseinheit (2) ein Alarmerzeugungsmodul (72) umfasst, das dazu in der Lage ist, einen Algorithmus anzuwenden, der eine oder mehrere Flugkontextinformationen und den Wert der globalen Anzeige verwendet, der vom Aggregationsmodul (70) bestimmt wurde, um zu bestimmen, ob ein Alarm ausgegeben werden muss.

## Claims

1. A system (1) for monitoring a pilot of an aircraft, in order to detect an incapacitation of the pilot, the aircraft being equipped with at least one piloting device (40) which can be manually actuated by the pilot, **characterized in that** the system (1) includes:
- the piloting device (40),
- at least one pressure sensor (62, 65) so as to generate an elementary indicator when a pressure exerted by the pilot on the piloting device is adapted to the use of this piloting device, the pressure sensor integrating:
a capacitive pressure sensor (11, 12) mounted on a surface of the piloting device (40), and,
a modeling module (51 to 59), the modeling module being adapted to compute the elementary indicator, from the signal delivered by the capacitive pressure sensor, by implementing a contact model that depends on the nature of the piloting device,
- a computing unit (2) programmed for processing the elementary indicator delivered by the pressure sensor and for determining a current state of the pilot and, when said current state corresponds to an incapacitation of the pilot, for issuing an alarm (ALARM).

2. The system according to claim 1, wherein the pressure sensor is a contact pressure sensor suitable for detecting the contact of the pilot's hand on the piloting device.

3. The system according to claim 1 or claim 2, wherein the pressure sensor is a force pressure sensor suitable for detecting the application of a force by the pilot, on the piloting device.

4. The system according to any of claims 1 to 3, wherein the computing unit (2) receives at least one item of flight context information (Info_context) from an auxiliary system (31 to 34) equipping the aircraft, and wherein the computing unit (2) processes the elementary indicator delivered by the pressure sensor taking into account said flight context information.

5. The system according to claim 4, wherein the flight context information (Info_context) is a piece of information on a flight phase of the aircraft or a value of at least one flight parameter of the aircraft, the flight phase information being selected among: a taxiing phase, a take-off phase, a cruising phase, and a landing phase, and the flight parameter information being selected among: a height, a speed, an engagement status of the autopilot, a position of a noise wheel steering control, a position of a throttle, a position of a stick, and a position of a rudder-bar.

6. The system according to any of claims 1 to 5, including a plurality of pressure sensors and an aggregation module suitable for aggregating (70) all or part of the plurality of indicators delivered by the plurality of pressure sensors into a global indicator as a current state of the pilot.

7. The system according to claim 6, wherein the aggregation module (70) selects the elementary indicators to be taken into account for calculating the global indicator according to the flight context information.

8. The system according to claim 6 or claim 7, in which the computing unit (2) comprises an alarm generation module (72) capable of applying an algorithm using one or more items of flight context information and the value of the global indicator determined by the aggregation module (70) to determine whether to issue an alarm.
